# EUROPEAN PATENT SPECIFICATION

(11) **EP 0 883 380 B1**
(45) Date of publication and mention of the grant of the patent: **29.08.2001**
(21) Application number: 97905934.2
(22) Date of filing: 12.02.1997
(51) Int. Cl.: A61B 17/62, B29D 31/00, B32B 31/00

(54) **EXTERNAL FIXATION DEVICE WITH COMPOSITE RINGS**
EXTERNE BEFESTIGUNGSVORRICHTUNG MIT RINGEN AUS KOMPOSITMATERIAL
DISPOSITIF DE FIXATION EXTERNE A CERCLAGE COMPOSITE

(30) Priority: 22.02.1996 US 605563
(43) Date of publication of application: 16.12.1998
(73) Proprietor: DEPUY ORTHOPAEDICS, INC., Warsaw, Indiana 46581-0988 (US)
(72) Inventor: ALLOTT, Jeffrey, Gibson, Elizabethtown, NY 12932 (US); STURGEON, Donald, Martin, Wilmington, DE 19806 (US)
(74) Representative: Jones, Alan John
(86) International application number: US9702250
(87) International publication number: WO9730651

(56) References cited:
- EP-A- 0 258 102
- WO-A-87/04916
- DE-A- 3 935 134
- US-A- 4 591 155
- US-A- 5 062 844
- US-A- 5 275 598

## Description

### Background of the Invention:

The present invention relates to fiber-reinforced composite structures having improved performance under shear, bending, and torsional stresses. The composite structures are especially useful as external fixation devices.

External fixation methods for treating bone fractures are well-known in the art, including the use of a number of curved rings or curved partial rings that are spaced apart and structurally connected using a plurality of tie rods. Transversely extending pins or wires are attached to the rings and extended from the rings and into the bones so that the frame and transverse pins support and/or load the bone tissue in a desired manner. Such methods are described in Fischer U.S. Patent No. 4,308,863 and Jamison et al. U.S. Patent No. 5,062,844 as well as German Publication No. DE 3935134-A.

External fixation ring systems are commercially available and utilize either metal or composite rings. Composite rings that are transparent to x-rays are desirable as they do not interfere with evaluation of medical radiographs. In addition, composite rings are lighter than comparable metal rings, resulting in improved patient comfort.

The above-referenced Fischer Patent describes partial metal rings having an I-beam cross-section resulting in high strength with minimum weight. Although I-beam structures generally make efficient use of materials by using the flange sections to resist bending forces, they generally provide more limited resistance to torsional forces.

The above-referenced German Publication describes a solid plastic core reinforced by fibers on surface of core.

In use, external fixation rings are subjected to shear, torsional, and bending forces. External fixation devices for immobilizing a bone fracture that include external fixation rings in the form of the fiber-reinforced composite structures according to the current invention provide an improved balance of mechanical properties for improved performance under combined bending, shear, and torsional forces.

The external fixation device of the present invention is set forth in Claim 1. Preferred embodiments are set forth in Claims 2 to 7.

### Brief Description of the Drawings:

Fig. 1 is a perspective view of an external fixation device of this invention installed on a fractured bone illustrating the external fixation rings;
Fig. 2 is an exploded perspective view partially in section of an external fixation ring;
Fig. 3 is a plan view of fixation rings of 240 degrees and 120 degrees angular lengths connected by connecting links; and
Figs. 4A-4G illustrate a preferred process for making the fixation ring.

### Detailed Description of the Preferred Embodiment:

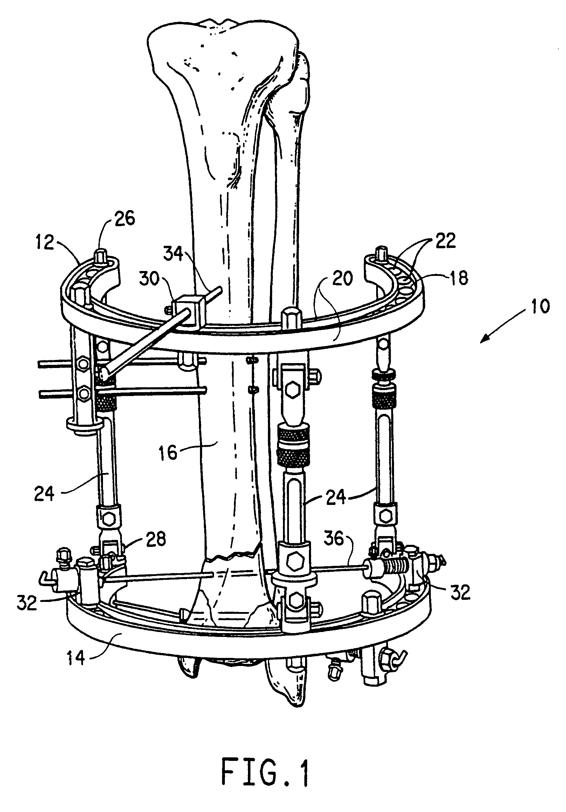

The embodiment chosen for purposes of illustrating the present invention is shown in Fig. 1. The external fixation device 10 is composed of arcuate fixation rings 12 and 14, which are disposed about a fractured bone 16. The fixation rings 12 and 14 have an I-beam cross-section composed of a central web portion 18 and a pair of flanges 20. A plurality of holes 22 are formed and symmetrically spaced in the web portion. When installed upon fractured bone 16, a plurality of tie rods 24 pass through some of the holes 22 and are attached to rings 12 and 14 by fasteners 26 and 28. Pin holders 30 and 32 are fastened at various locations on rings 12 and 14 and mount pins 34 and 36, which pass through the bone 16.

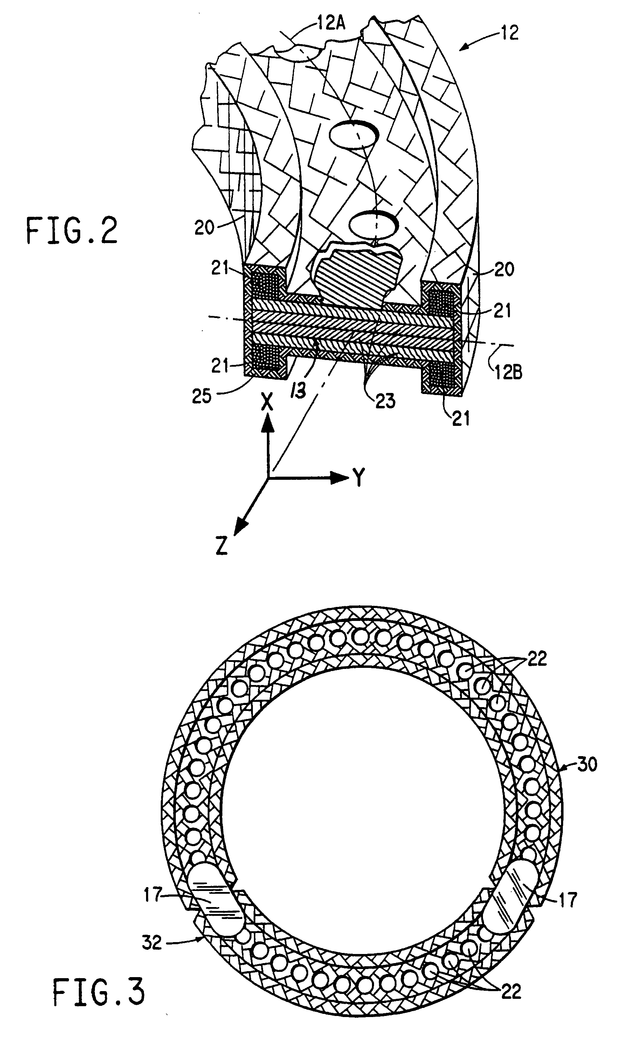

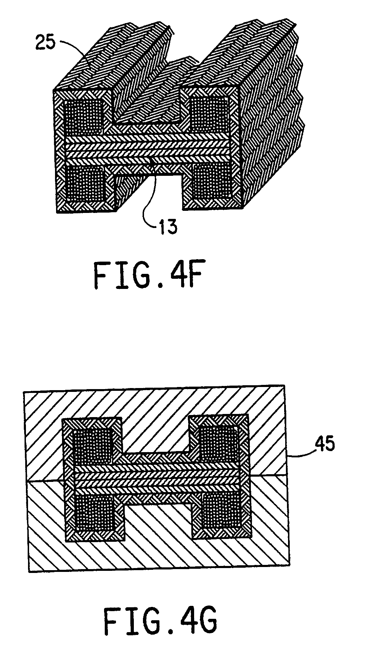

As best shown in Fig. 2, a fixation ring 12 has a core (shown as core 13 in Fig. 4F) formed of unidirectional fibers 21 in the flanges 20 and a plurality of layers 23 of unidirectional fibers in the web and a braided sleeve 25 formed about the core 13. The sleeve 25 is bound to the core 13 by resin, which impregnates the fiber of the core and the sleeve. The ring has a longitudinal axis designated 12A.

The unidirectional fibers 21 are aligned along the curved flanges 20, approximately parallel to the axis 12A of the web core. Use of unidirectional fibers in the flanges 20 of the core results in improved resistance to bending forces in the final composite. Preferably, the unidirectional fibers 21 are oriented between 0 and about ± 15 degrees relative to the axis of the core, 12A in Fig. 2.

The web portion of the core is preferably reinforced with fiber layers 23 oriented roughly parallel (between zero and about ± 15 degrees) to unidirectional fibers 21 and said layers 23 containing fibers oriented between approximately ± 45 degrees ± 15 degrees relative to a tangent to the longitudinal axis of the core, more preferably, between approximately ± 45 degrees ± 5 degrees, most preferably, approximately ± 45 degrees. This provides optimum resistance to shear stresses, which are generally greatest in the interior of the core. Preferably, the fiber orientation in the interior of the core is symmetric about the cross-sectional center-line 12B and balanced in the positive and negative directions.

In order to improve the overall resistance of the composite ring to torsional forces and delamination within the core, a layer of fiber material is applied to the exterior surface of the inner core (e.g., braided sleeve 25). The fiber material is applied such that it conforms to the outer shape of the core and is oriented at between approximately ± 45 degrees ± 15 degrees relative to the tangent of the longitudinal axis through the centroid of the ring or core, more preferably, between approximately ± 45 degrees ± 5 degrees, most preferably, approximately ± 45 degrees.. The term "relative to the tangent of the longitudinal axis through the centroid of the ring or core" means that for a particular cross-section, when XYZ coordinate axes at the centroid of the cross-section are drawn and the cross-section lies in the X-Y plane and Z is perpendicular to the X-Y plane, Z is the tangent to the longitudinal axis 12A in Fig. 2 for that cross-section. All angles (for that cross-section) are measured relative to the Z axis. For horizontal surfaces, the angles are measured in the Y-Z plane and for vertical surfaces, angles are measured in the X-Z plane.

A wide range of fibers and polymeric matrices may be used to form the composite rings. For external fixation devices where high strength and stiffness are required, carbon-fiber reinforced epoxy resins are preferred. Other fibers including aramid, glass, or nylon may be also used. Preferably, continuous filament reinforcing fibers are used. Thermoset or thermoplastic matrices may be used, including polyester, nylon, or bismaleimide resins. Preferably, the composite device comprises approximately 30-70 vol% fiber and 70-30 vol% resin. More preferably, the composite comprises approximately 50-70 vol% fiber, most preferably, approximately 65 vol% fiber.

External fixation rings may be formed from the composite rings in a variety of diameters, lengths, and cross-section dimensions. For rings of I-beam cross-sections, increasing the thickness of the flange in general increases the bending strength; shear strength may be increased by increasing the thickness of the web. The external fixation devices may be formed as partial rings, for example, as 120 degrees (1/3 ring), 240 degrees (2/3 ring), 180 degrees (1/2 ring), or full rings. External fixation rings generally include a plurality of holes 22 symmetrically spaced along the length of the arcuate frame. The apertures may be drilled into the part during final machining.

As shown in Fig. 3, partial ring segments 30 and 32 may be joined together by the use of connecting links 17. The connecting link 17 may include bosses (not shown) to engage the ring between the flange sections for rings having I-beams cross-sections. The link is attached to the ring using screws (not shown) through holes 22 at the ends of each ring section.

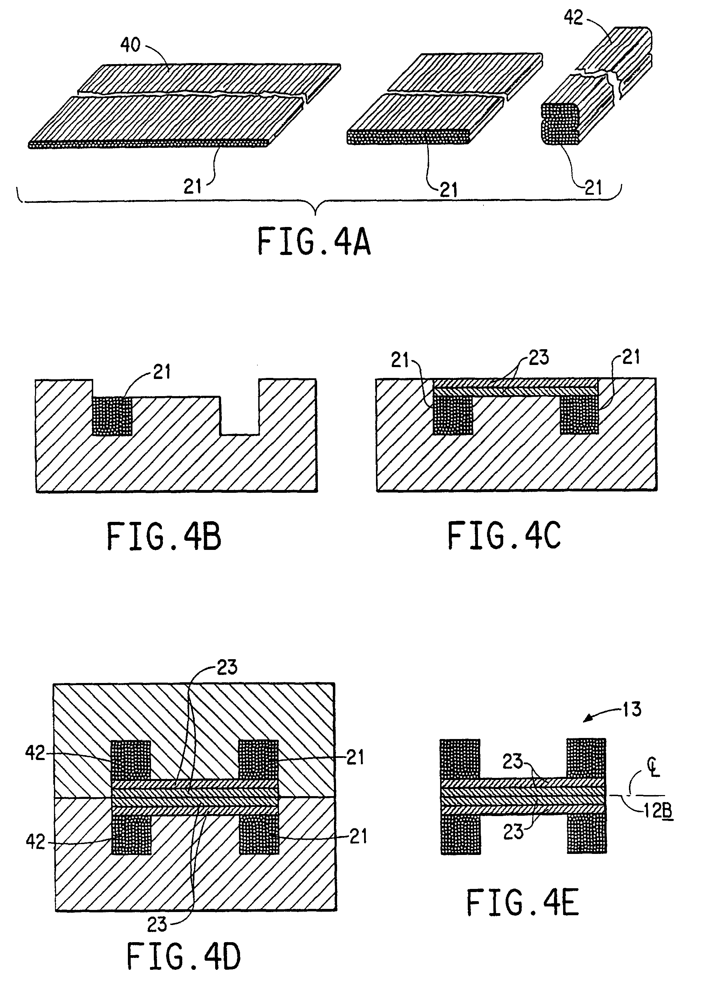

Figs. 4A-4G illustrate a process for forming an arcuate fiber-reinforced composite I-beam ring 12. An arcuate fiber-reinforced resin I-beam core is formed, covered with a fiber material, and enclosed in a mold shaped to conform to the shape of the fiber-covered core. Resin is then injected into the closed mold under pressure to impregnate the fiber material and bond it to the core. Injecting resin under pressure into the unimpregnated fiber material surrounding the core results in isobaric conditions during injection and more uniform properties in the final part than would be achieved by applying prepreg material to the core and consolidating under heat and pressure with no resin injection.

Figs. 4A-4E illustrate steps in forming the arcuate fiber-reinforced composite core having an I-beam cross-section. Unidirectional fiber prepreg material (containing unidirectional fibers and resin) may be placed in grooves in each half of a mold, as shown in cross-section for one half of the mold in Fig. 4B. To facilitate lay-up of the unidirectional fibers 21, unidirectional fiber prepreg sheet material may be cut into strips 40 with the fibers running along the long dimension of each strip. The strips may then be folded along the direction of the unidirectional fibers 21 several times to provide unidirectional "noodles" 42. In Fig. 4A, the strips have been folded in half and then in thirds to form a noodle structure. The noodles are placed in grooves which run along the inner and outer edges of each side of the mold 30 and will ultimately form the flange section 20 of the core 13. Alternatively, layers of unidirectional tow that has been impregnated with resin may be laid into the groove. However, the use of prepreg sheet material assures more reproducible lay-up of fibers and better control of fiber orientation in the final part.

A convenient way to form the web portion of the core is to cut a plain weave prepreg fabric at 45 degrees to the fiber axes. Plain weave is preferred because there are equal amounts of fiber in the warp and fill directions. Other weaves may be used which do not contain balanced fiber ends in the warp and fill directions. The fiber ends in the + and - directions may then be balanced by flipping the fabric over in alternating layers or by cutting alternating layers at 90 degrees from each other in the original fabric. Alternatively, unidirectional fiber prepreg sheet may be used with alternating layers of fiber oriented at approximately 45 degrees in the + and - directions. Woven fabrics are generally more convenient when constructing parts having substantial thickness as they are generally available in greater thicknesses and contain more fiber ends than commercially available unidirectional ply materials. The undulations present in woven fabric can result in parts having higher toughness as the undulations may resist the propagation of cracks. However, the undulations may also reduce the strength and stiffness of the final part.

The cut-to-size rectangular prepreg strips or layers 23 are placed into each of the mold halves overlaying the unidirectional noodles at the inner and outer edges as shown in Fig. 4C. To facilitate working the fabric strips into the shape of curved molds, the mold may be warmed during placement of the fabric plies. To increase the thickness of the web, the layers could be also placed in between the noodles.

After lay-up of the noodles of unidirectional fibers 21 and fabric layers 23, the mold is closed as shown in Fig. 4D and heated under pressure to sufficient temperature and pressure to consolidate the core 13. The consolidated core 13 (Fig. 4E) is then removed from the mold, and may be machined to the appropriate dimensions and the surface sanded to remove excess resin and mold lines. Preferably, the surface is sanded to provide an even matted finish with no visible fiber fraying. This is conveniently done using a sandblaster, taking care not to oversand.

As shown in Fig. 4F, a fiber material is then applied to the outer surface of the prepared inner core. This may be done by pulling one or more ± 45 degrees braided sleeves 25 having a circumference that is approximately equal to that of the composite I-beam over the outer surface of the core. By using a braid having a circumference that is approximately equal to that of the core, the fibers in the braid will be oriented approximately ± 45 degrees relative to the tangent to the longitudinal axis of the core. The initial braid angle is preferably chosen such that the final braid angle in the composite part is approximately ± 45 degrees. Depending on the weight of braid used, a single braided sleeve may be used, or multiple sleeves may be applied to achieve the desired final part volume. In general, the number of filaments in the tow and the pick count should be chosen to reduce the number of undulations in the fabric for a desired weight. Braided fabrics are especially useful as the outer surface fiber material because they can be easily worked into and around complex shapes such as I-beams and eliminate seams in the outer covering fibrous material.

Alternatively, the fiber material may be applied to the inner core by overbraiding or overwinding the part with a tow of the desired fiber. Tow may be wound on the part, alternating layers of approximately + 45 degrees and - 45 degrees relative to the tangent of the arc of the core. This approach is more readily applied to simple shapes such as rectangular, square, or circular cross-sections.

After application of the fiber material to the outer surface of the inner core, the part is placed into an injection mold 45 (Fig. 4G) shaped to conform to the shape of the covered inner core. The mold is closed, heated, and resin injected under pressure to impregnate the braided preform to form a composite article.

## Claims

1. An external fixation device (10) for immobilizing a bone fracture that includes a plurality of external fixation rings (12, 14), a plurality of pins (34, 36) anchored to the bone (16) and clamped to the fixation rings (12, 14) and lockable adjustment rods (24) interconnecting the fixation rings (12, 14), **characterised in that** said fixation rings (12, 14) are a unitary composite structure that includes a core (13) of a resin-impregnated fiber matrix, a fiber material sleeve (25) covering and bound to said core (13) by resin impregnating said fiber material sleeve (25), the core (13) including a curved I-beam cross-section having flanges (20) and a web (18), said core fibers including unidirectional fibers (21) within said flanges (20) and further including layers (23) of material forming said web (18).

2. The external fixation device of claim 1, the fixation rings (12, 14) being unitary composite structures wherein the core fibers are oriented in transverse and non-transverse directions with respect to the longitudinal axis (12A) of the article.

3. The external fixation device of claim 1, wherein said fiber material sleeve (25) covering said core (13) is a braided sleeve.

4. The external fixation device of claim 2, wherein said fiber material sleeve (25) covering said core (13) is a braided sleeve.

5. The external fixation device of claim 1, wherein said layers (23) of material include fibers, said fibers within said layer being oriented between about ± 45 degrees ± 15 degrees relative to a tangent to the longitudinal axis (12A) of the core, the angles being balanced in the positive and negative directions.

6. The external fixation device of claim 5, wherein said fiber material covering said core (13) comprises braided or wound continuous filament tow and wherein the braid or winding angles are between about ± 45 degrees ± 15 degrees relative to the tangent of the longitudinal axis (12A) through the centroid of the core (13).

7. The external fixation device of claim 1, wherein said fiber material are carbon fibers and said resin material is epoxy resin.

## Patentansprüche

1. Externe Befestigungsvorrichtung zur Immobilisierung eines Knochenbruches, welche eine Vielzahl äußerer Fixierungsringe (12, 14), eine Vielzahl am Knochen (16) verankerter und an den Fixierungsringen (12, 14) geklemmter Stifte (34, 36) sowie die Fixierungsringe (12, 14) verbindende verriegelbare Einstellstangen (24) aufweist, **dadurch gekennzeichnet**, daß die Fixierungsringe (12, 14) einteilige Verbundstrukturen sind, die einen Kern (13) aus einer kunstharzgetränkten Fasermatrix und eine Hülse (25) aus Fasermaterial aufweisen, welche den Kern (13) umhüllt und durch Kunstharztränkung der Hülse (25) aus Fasermaterial mit diesem verbunden ist, wobei der Kern (13) einen gekrümmten I-Träger-Querschnitt mit Flanschen (20) und einem Steg (18) umfaßt und in den Flanschen (20) in einer Richtung verlaufende Fasern sowie Lagen (23) aus Material, welche den Steg (18) bilden, aufweist.

2. Externe Befestigungsvorrichtung nach Anspruch 1, bei welcher die Fixierungsringe (12, 14) einteilige Verbundstrukturen sind, in welchen Kernfasern quer und nicht quer zur Längsachse (12A) des Erzeugnisses verlaufen.

3. Externe Befestigungsvorrichtung nach Anspruch 1, bei welcher die Hülse (25) aus Fasermaterial, welche den Kern (13) bedeckt, eine geflochtene Hülse ist.

4. Externe Befestigungsvorrichtung nach Anspruch 2, bei welcher die Hülse (25) aus Fasermaterial, welche den Kern (13) bedeckt, eine geflochtene Hülse ist.

5. Externe Befestigungsvorrichtung nach Anspruch 1, bei welcher die Lagen (23) aus Material aus Fasern bestehen, wobei die Fasern in der Lage in einem Bereich zwischen etwa ± 45 Grad und ± 15 Grad relativ zu einer Tangente an der Längsachse (12A) des Kernes ausgerichtet sind undf die Winkel in positiver und negativer Richtung im Gleichgewicht zueinander stehen.

6. Externe Befestigungsvorrichtung nach Anspruch 5, bei welcher das den Kern (13) bedekkende Fasermaterial einen durchgehend geflochten oder gewickelten Faserstrang umfaßt, wobei die Flecht- bzw. Wickelwinkel zwischen etwa ± 45 Grad und ± 15 Grad relativ zu einer Tangente an der Längsachse (12A) durch den Schwerpunkt des Kernes liegen.

7. Externe Befestigungsvorrichtung nach Anspruch 1, bei welcher das Fasermaterial aus Karbonfasern und das Kunstharzmaterial aus Epoxidharz besteht.

## Revendications

1. Dispositif (10) de fixation externe servant à immobiliser une fracture osseuse, lequel dispositif comprend une pluralité d'anneaux (12, 14) de fixation externe, une pluralité de broches (34, 36) fixées sur l'os (16) et fixées sur les anneaux de fixation (12, 14), et des tiges de réglage (24) verrouillables reliant entre eux les anneaux de fixation (12, 14), **caractérisé en ce que** lesdits anneaux de fixation (12, 14) sont une structure composite unitaire qui comprend une partie centrale (13) d'une matrice de fibres imprégnées de résine, une gaine (25) de matière fibreuse recouvrant ladite partie centrale (13) et collée à celle-ci par de la résine imprégnant ladite gaine (25) de matière fibreuse, la partie centrale (13) comprenant une section courbe en double T, ayant des membrures (20) et une âme (18), lesdites fibres de la partie centrale comprenant des fibres unidirectionnelles (21) dans lesdites membrures (20) et comprenant en outre des couches (23) de matière formant ladite âme (18).

2. Dispositif de fixation externe selon la revendication 1, dans lequel les anneaux de fixation (12, 14) sont des structures composites unitaires où les fibres de la partie centrale sont orientées suivant des directions transversales et non transversales par rapport à l'axe longitudinal (12A) de l'article.

3. Dispositif de fixation externe selon la revendication 1, dans lequel ladite gaine (25) de matière fibreuse recouvrant ladite partie centrale (13) est une gaine tressée.

4. Dispositif de fixation externe selon la revendication 2, dans lequel ladite gaine (25) de matière fibreuse recouvrant ladite partie centrale (13) est une gaine tressée.

5. Dispositif de fixation externe selon la revendication 1, dans lequel lesdites couches (23) de matière comprennent des fibres, lesdites fibres dans ladite couche étant orientées à environ ± 45 degrés ± 15 degrés par rapport à une tangente à l'axe longitudinal (12A) de la partie centrale, les angles étant équilibrés dans les directions positives et négatives.

6. Dispositif de fixation externe selon la revendication 5, dans lequel ladite matière fibreuse recouvrant ladite partie centrale (13) comprend de la filasse tressée ou de la filasse à filaments continus enroulés et dans lequel la tresse ou les angles d'enroulement sont à environ ± 45 degrés ± 15 degrés par rapport à la tangente à l'axe longitudinal (12A) traversant le centre de gravité de la partie centrale (13).

7. Dispositif de fixation externe selon la revendication 1, dans lequel ladite matière fibreuse est constituée de fibres de carbone et où ladite matière de résine est de la résine époxyde.
